# EUROPEAN PATENT SPECIFICATION

(11) **EP 2 429 457 B1**
(45) Date of publication and mention of the grant of the patent: **12.10.2016**
(21) Application number: 10727873.1
(22) Date of filing: 07.05.2010
(51) Int. Cl.: A61F 2/38

(54) **IMPROVED ORTHOPAEDIC DEVICE**
VERBESSERTE ORTHOPÄDISCHE VORRICHTUNG
DISPOSITIF ORTHOPÉDIQUE AMÉLIORÉ

(30) Priority: 08.05.2009 IT BO20090291
(43) Date of publication of application: 21.03.2012
(73) Proprietor: Alma Mater Studiorum -Universita' di Bologna, 40126 Bologna (IT); Istituto Ortopedico Rizzoli, 40136 Bologna (IT)
(72) Inventor: PARENTI CASTELLI, Vincenzo, 40141 Bologna (IT); CATANI, Fabio, 40054 Budrio (IT); SANCISI, Nicola, 40138 Bologna (IT); LEARDINI, Alberto, 40128 Bologna (IT)
(74) Representative: Puggioli, Tommaso
(86) International application number: PCT/IB2010/052024
(87) International publication number: WO 2010/128485

(56) References cited:
- WO-A-2007/074387
- US-A- 3 969 773
- US-A- 5 413 604

## Description

### Technical Field

This invention relates to an improved orthopaedic device and, in particular, to an artificial orthopaedic device designed to assist or replace damaged joints.

### Background Art

Prior art artificial orthopaedic devices, broadly divided into external prostheses or limb prostheses, braces or orthotics, and internal prostheses or joint prostheses, are widely used in the field of medical surgery.

In joints (meaning here any generic joint although, for convenience, reference is preferably made to the knee joint), there is contact between joint surfaces that have motion relative to each other.

This relative motion is substantially defined by relative rolling motion and relative sliding motion between the joint surfaces.

The relative motion is guaranteed by joint structures such as ligaments, muscles and cartilaginous structures which keep the joint surfaces in the correct relative position.

When the joint surfaces suffer damage as a result of a degenerative disease, internal prostheses may be used to recover joint functions.

Joint prostheses attempt to replicate and replace the degenerated natural joint surfaces using cams, working surfaces and intermediate elements (such as plastic inserts) that reproduce the relative movement of the natural joint.

In particular, replication of the original movement relies partly on the shape of the mating surfaces that come into contact in the prosthesis, these surfaces being constructed to emulate the typical shape of the natural surfaces, and partly on the natural joint structures that are not removed during the surgical operation necessary to implant the prosthesis.

Indeed, it should be noted that during implantation of a joint prosthesis, the main joint structures at the joint to be replaced - for example, the cruciate ligaments in the case of knee prostheses - are very often cut to allow the prosthesis to be inserted.

In many cases, the prosthesis is inserted in such a way that the conditions for keeping the optimum relative position between the joint surfaces are lost.

Generally speaking, in most prior art prostheses, the mating surfaces that come into contact during movement tend to slide against each other, leading to rapid wear caused by the resulting friction and high specific loads.

To overcome these drawbacks, prostheses such as the ones described in international patent application WO2007/074387 have been developed. These are based on the special shape of the mating surfaces. In particular the mating working surfaces described in that international patent application are either defined as portions of ruled surfaces with curvilinear directrix having as directrices the polar coordinates of a planar relative motion or are made to coincide with the axoidal surfaces of a spherical relative motion.

The 1-degree-of-freedom planar or spherical relative motion is an approximation of the real spatial motion of the natural joint, so that the relative motion between the axoidal surfaces is a rolling only motion, without sliding.

A prosthesis of the kind described in the above mentioned application WO2007/074387, however, especially in the case of knee prostheses, requires excessive removal of bony material from the femur.

Further, and different, types of prior art prostheses, are described in documents US-A-5413604, WO2007/119173 and US-A-3969773.

Document WO2007/119173 describes a prosthesis comprising a spherical joint with three degrees of freedom positioned at the centre of the medial condyle and combined with a track that does not constrain prosthesis movement along a set path. The prosthesis does not therefore precisely approximate the one-degree-of-freedom motion of a natural knee joint and does not completely recreate the anatomical constraints of the joint, especially in cases where the anatomical knee structures are damaged or have been cut or removed to allow the prosthesis to be inserted. As a result, the joint is less controlled than under natural conditions, creating a relative movement which is less anatomical and at the same time placing an unnatural load on the other parts of the knee structures.

The prosthesis described in document US-A-5413604, comprising a spherical joint with three degrees of freedom positioned at the centre of the lateral condyle, also defines a movement with too many degrees of freedom.

Document US-A-3969773 relates to a prosthesis structured in such a way as to define a planar movement between two circular surfaces whose centres are always equidistant and joined by two rigid members and/or by two links.

These two surfaces effectively roll on one another but the planar motion created according to the proposed solution does not allow real joint motion to be replicated with sufficient precision.

Furthermore, the two links do not fully constrain the rotation of the prosthesis about the common normal of the contact surfaces and the additional connecting members are needed which, however, make it essential to choose circular rolling surfaces.

### Disclosure of the Invention

In this context, the main technical purpose of this invention, which is defined in claim 1, is to propose an orthopaedic device that is an improvement over prior art devices.

The aim of this invention is to provide an orthopaedic device that can replicate as closely as possible the natural movement of the joint replaced and that is also easy to implant.

### Brief Description of the Drawings

Further features and advantages of the invention are more apparent in the non-limiting description which follows of a preferred but non-exclusive embodiment of an orthopaedic device as illustrated in the accompanying drawings, in which:
- Figure 1 is a schematic representation of the knee joint functional to the definition of a prosthesis according to this invention;
- Figure 2 illustrates a principle diagram of a knee prosthesis according to the invention in a schematic front view;
- Figure 3 is a schematic front view illustrating a first preferred embodiment of a prosthesis according to the invention;
- Figure 4 is a schematic front view illustrating a second preferred embodiment of the orthopaedic device according to the invention;
- Figure 5 is a schematic front view illustrating a third preferred embodiment of the orthopaedic device according to the invention;
- Figure 6 is a schematic perspective view of a detail of the prosthesis of Figure 5;
- Figure 7 is a schematic front view illustrating a fourth preferred embodiment of the orthopaedic device according to the invention;
- Figure 8 schematically represents a detail of the orthopaedic device of Figure 7.

### Detailed Description of the Preferred Embodiments of the Invention

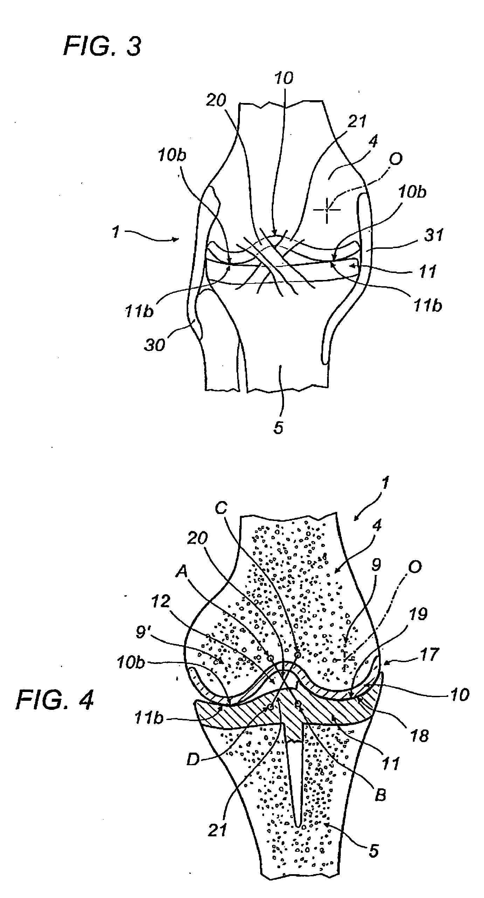

With reference to the accompanying drawings, in particular Figures 3, 4, 5 and 7, the numeral 1 denotes an orthopaedic device or, generally speaking, a prosthesis according to this invention.

As described in patent application WO2007/074387, the motion of a joint can be approximated to a spherical motion, that is to say, it is referable to a succession of instantaneous rotations about a variable directional axis that passes through a point fixed to the two members.

Generally speaking, a spherical motion defined by a spherical pair has three degrees of freedom. However, the spherical motion that approximates the motion of the knee, which this specification refers to in particular but without limiting the scope of the invention, has only one degree of freedom.

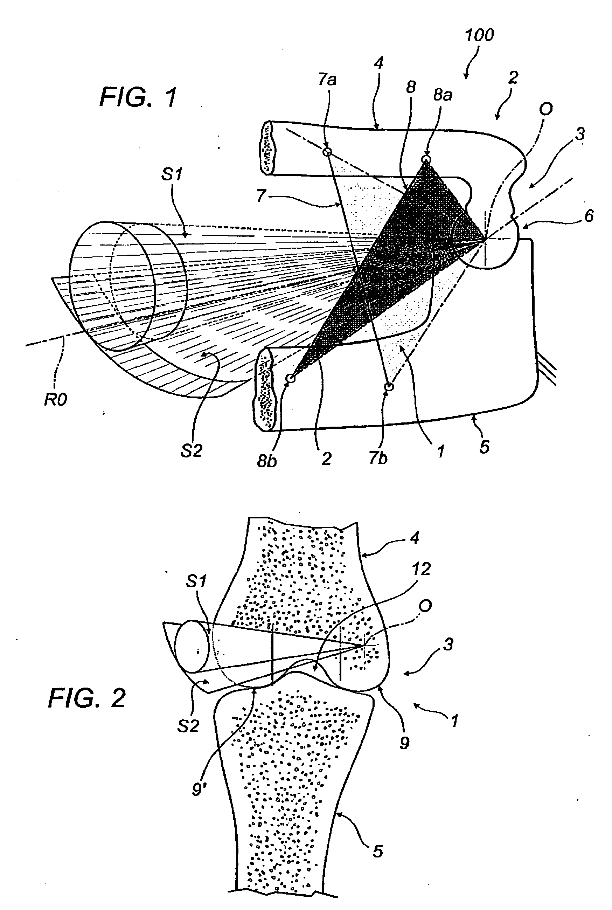

As illustrated in Figure 1, a joint 2 can therefore be schematized by and made referable to an equivalent spatial mechanism with one degree of freedom.

By way of non-limiting example, a knee 3 (Figure 1) can be basically referred to a model 100 comprising a first bone segment 4 coupled to a second bone segment 5 by a spherical kinematic pair 6.

The real motion of the knee 3 is constrained by the natural anatomical ligamentous structures such as the cruciate ligaments and the collateral ligaments so that the approximating spherical motion can be said to have only one degree of freedom.

For simplicity, the description below refers to a spherical pair to mean a spherical kinematic pair or a kinematic pair which allows three degrees of freedom in space, that is to say, rotations about three axes passing through the centre of the pair itself.

The bone segments 4 and 5 are constrained to each other by a first rod 7 and a second rod 8.

The first rod 7 is connected to the first bone segment 4 at a first anchor point 7a and to the second bone segment 5 at a second anchor point 7b.

The second rod 8 is connected to the first bone segment 4 at a first anchor point 8a and to the second bone segment 5 at a second anchor point 8b.

The first rod 7 and the second rod 8 of the model 100 represent the main natural anatomical ligamentous structures.

By way of a specific, non-exclusive and hence non-limiting example, the first rod 7 and the second rod 8 may represent in the model 100 the two cruciate knee ligaments. In a further specific, non-exclusive and hence non-limiting example, the first rod 7 and the second rod 8 may represent in the model 100 one cruciate ligament and one collateral ligament.

The representation of the main ligamentous structures in the model 100 by means of the rods 7 and 8 allows the isometry which these structures present during natural joint motion to be recreated in the spherical model 100 and in the motion it creates.

The one-degree-of-freedom spherical motion created by means of the model 100 thus makes it possible to approximate natural knee joint motion and to recreate the isometry at least of the main ligamentous structures of the joint.

The helical axis, or axis R0 of instantaneous rotation, of the one-degree-of-freedom spherical motion that approximates the motion of the knee joint is the axis defined by the intersection of a first plane π1, identified by the centre "O" of the spherical pair 6 and the two points 7a, 7b anchoring the first rod 7 to the bone segments 4, 5, with a second plane π2, identified by the centre "O" of the spherical pair 6 and the two points 8a, 8b anchoring the second rod 8 to the bone segments 4, 5.

The axis R0 of instantaneous rotation, while continuing to pass through the fixed point "O" during relative motion, changes its direction as a result of the relative motion between the members 4 and 5.

With reference to Figure 1, the geometric locus of the positions occupied by the axis R0 of instantaneous rotation during the motion is a first conic surface S1 for the first member 4 and a second conic surface S2 for the second member 5.

In other words, the first and second surfaces S1, S2 are ruled surfaces with curvilinear directrix in which the generator R0 moves on a corresponding curvilinear directrix passing through a fixed point during the relative motion of the members 4 and 5 (point "O", Figure 1).

The first and second surfaces S1, S2 are the axoids of the spherical motion referable to the motion of the joint 2.

The motion of the joint 2 is in practice referred to a succession of instantaneous rotations about a variable directional axis passing through the fixed point "O".

The motion of the joint 2 is fully defined by the rolling of the second conic surface S2 on the first conic surface S1.

It should be noted that the two axoidal conic surfaces S1, S2 of the spherical motion have the same vertex and touch at every instant along a generator that coincides with axis R0 of instantaneous rotation at a given instant.

Note that during such motion, the axoidal surfaces of the spherical motion rotate on each other without sliding.

The axoidal surfaces S1, S2 of the motion are inferred from the spatial model 100 used as the mechanism equivalent to the joint 2.

With reference to Figure 2, considering as preferred embodiment the knee joint 3, the first bone segment 4 is the femur while the second bone segment 5 is the tibia.

The axoids S1, S2 of the motion are positioned as shown in Figure 2 and the fixed centre "O" must be considered as being substantially positioned at the medial condyle 9 of the femur 4.

As illustrated, the centre "O" is located at a medial position relative to the sagittal midplane of the knee.

In more detail, the positioning of the point "O" is an optimization of the data obtained experimentally on samples defining the spherical motion that simulates the real general motion of the natural knee.

A first preferred embodiment of an orthopaedic device 1 according to this invention, illustrated in Figure 3, comprises a first component 10 designed to be rigidly coupled to the femur 4 and a second component 11 designed to be rigidly coupled to the tibia 5.

As illustrated, the components 10 and 11 comprise working or load surfaces 10b and 11 b in contact with each other.

Since the motion of the knee joint 3 is approximated with a one-degree-of-freedom spherical motion, the load surfaces 10b, 11b are preferably generated by means of the axoidal conic surfaces S1, S2 of the spherical motion.

More specifically, the surfaces 10b, for example in the form of replicas of the natural condyles are attached to the axoidal surface S1.

The rotation of the surface S1 on the surface S2, being axoids of the motion, generates the surfaces 11b associated with the surface S2 itself.

Alternatively, the load surfaces 10b are preferably in the form of portions of conic surfaces with curvilinear directrix with vertex at "O", approximating the natural condyles.

Advantageously, the load surfaces 11b generated using the procedure described above are also portions of conic surfaces with curvilinear directrix with vertex at "O".

In other words, the load surfaces 10b, 11b are in the form of ruled conic surfaces.

The ruled conic surfaces 10b, 11b therefore touch along a segment, are more constrained than any other surfaces and the contact pressures are lower than those of generic contact surfaces.

Advantageously, according to the same principle, the procedure can be followed in reverse to build the tibial plate (surface 11b) and to generate the mating surface 10b corresponding to the condyles of the femur.

The mating load surfaces 10b, 11b therefore serve mainly to transmit the forces between the bone segments 4, 5.

The component 10 of the prosthesis 1 that can be associated with the femur 4 therefore comprises the load surface 10b and, symmetrically, the component 11 of the prosthesis 2 that can be associated with the tibia comprises the surface 11b which mates with the first load surface 10b of the first component 10.

In the configuration illustrated, the motion of the knee 3 is constrained by the natural anatomical ligamentous structures such as the cruciate ligaments, labelled 20 and 21, and the collateral ligaments, labelled 30 and 31, so that the approximating spherical motion has only one degree of freedom.

In practice, the relative motion of the components 10 and 11 occurs as if the components were constrained by a spherical pair and the degree of freedom limited to one by the "soft" or ligamentous natural anatomical structures.

In a second preferred embodiment, illustrated in Figure 4, the orthopaedic device 1 according to the invention comprises, again, a first component 10 designed to be rigidly coupled to the femur 4 and a second component 11 designed to be rigidly coupled to the tibia 5.

The orthopaedic device 1 according to the invention also comprises a spherical pair 17 to constrain the femur 4 and the tibia 5.

The spherical pair 17 is centred at the above mentioned fixed point "O" located in the condyle 9 of the femur.

In the second preferred embodiment illustrated by way of example, the spherical pair 17 comprises a spherical socket 18 formed in the component 11 and in which a spherical portion 19 formed on the first component 10 moves.

In other words, the convex spherical portion 19 fits into the spherical concavity of the socket 18.

This type of coupling provides three degrees of freedom between femur 4 and tibia 5 and two of these must be eliminated to obtain a spherical motion with one degree of freedom.

Advantageously, the orthopaedic device illustrated in Figure 4 comprises a first and a second rod, or connecting element, 20, 21 fastened to the first and second bone segments 4, 5 or to the first and second components 10, 11.

It should be noted that the connecting elements 20, 21 are preferably embodied by natural, artificial or biological ligament portions.

The rods 20 and 21 are provided for guiding the relative motion of the components 10 and 11, that is to say, for guiding the device 1.

Looking in more detail, four anchor points A, B, C, D were found experimentally to act in combination with the spherical pair 17 to define the one-degree-of-freedom spherical motion allowing the orthopaedic device 1 to replicate the real motion of the knee 3.

The anchor points A, C are located on the femur 4, while the anchor points B, D are located on the tibia 5 and are arranged as shown in Figure 4.

As illustrated, the points A, B, C and D are located on either of the bone segments 4, 5 or on the components 10 and/or 11, without distinction.

The rod 20 is fastened to the femur 4 and to the tibia 5 at the anchor points A and B.

The rod 21 is fastened to the femur 4 and to the tibia 5 at the anchor points C and D.

Advantageously, the anchor points A, B are connected by the rod 20 in crossed manner to the anchor points C, D connected by the rod 21.

Preferably, the anchor points A, B, C and D are located at a cavity 12 that extends in the femur 4 itself between the two condyles 9, 9'.

The rods 20 and 21 go through the cavity 12 to cross each other and connect the respective anchor points.

Preferably, the rods 20, 21 are located at a median position in the joint 2.

Advantageously, as mentioned, the rods 20, 21 are preferably embodied by portions of ligament.

It is important to observe that the fastening rods 20, 21 preferably comprise natural cruciate ligaments when the latter are not at risk or damaged.

Generally speaking, the rods 20, 21 are anchored to the femur 4 and to the tibia 5 in substantially the same way as the natural cruciate ligaments.

The ligament portions react only to traction and therefore, to prevent them from slackening and for replicating the natural motion, the orthopaedic device 1 comprises load surfaces 10b and 11b.

The load surfaces 10b and 11b, of the type described above, are modelled in the first component 10 and in the second component 11, respectively, on the anatomical side opposite the spherical pair 17 with respect to the rods 20 and 21.

Preferably, the first load surface 10b is associated with the bone segment 4 by the component 10 at the condyle 9' whose shape it matches.

In other words, the surface 10b is built substantially like the natural condyle 9' and the surface 11b is preferably generated as a mating surface of the surface 10b according to the above mentioned one-degree-of-freedom spherical motion.

Alternatively, the surface 10b is built like a conic surface portion with curvilinear directrix, having vertex at "O" and approximating the natural condyle 9'.

The surface 11b is generated substantially as a mating surface of the surface 10b according to the above mentioned one-degree-of-freedom spherical motion.

In practice, as illustrated in Figure 4, the orthopaedic device 1 comprises a spherical pair 17 at the first condyle 9, the load surfaces 10b, 11b at the second condyle 9' and the rods 20, 21 between the first and the second condyle 9, 9', at the cavity 12.

This embodiment of the device 1 makes it possible to save healthy parts of the joint 2, such as, for example, cruciate ligaments, without necessarily having to cut or manipulate the entire joint area.

The prosthesis 1 thus has a spherical coupling, commonly known as "ball and socket joint" and two ligaments used to constrain its motion and reduce the degrees of freedom from three to one.

Since these ligaments and the ball and socket cannot support high compression loads, the prosthesis 1 comprises mating load surfaces to bear the loads without impeding the motion of the device 1.

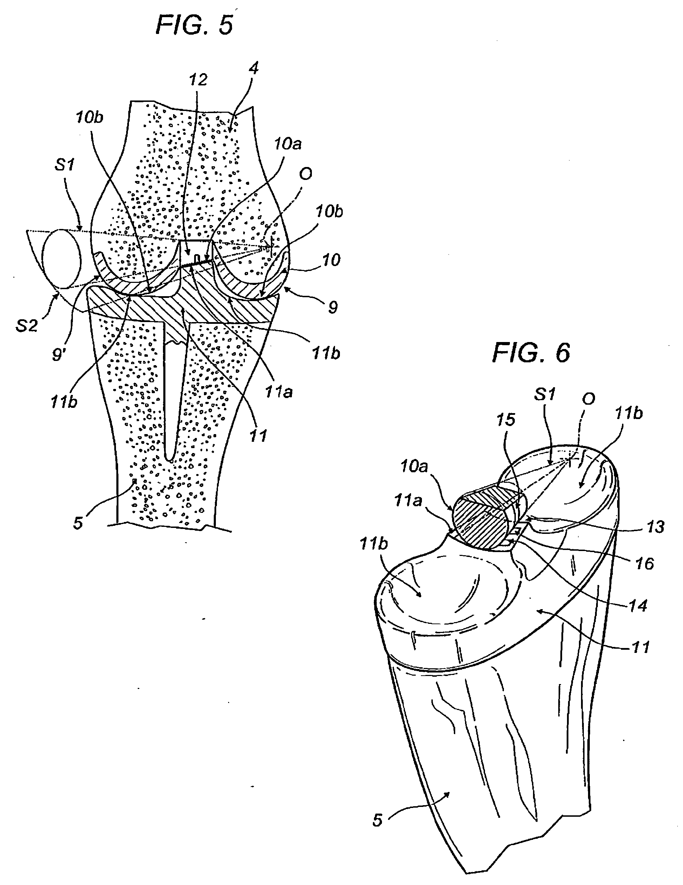

A third preferred embodiment of an orthopaedic device 1 according to the invention, illustrated in Figures 5 and 6, comprises, again, a first component 10 designed to be rigidly coupled to the femur 4 and a second component 11 designed to be rigidly coupled to the tibia 5.

The first and second components 10 and 11 comprise a respective device 1 guide and/or contact surface 10a, 11a which work in contact with each other.

In other words, the surfaces 10a and 11a are the surfaces of the components 10 and 11 which come into contact with each other and which are designed to guide the orthopaedic device 1.

The surfaces 10a and 11a are embodied as portions of the above mentioned conic surface S1 and of the above mentioned conic surface S2, respectively.

Advantageously, the components 10 and 11 are made in such a way that the contact surfaces 10a, 11a for guiding the joint are positioned between the two femoral condyles 9, 9'.

In practice, the surfaces 10a and 11 a are made by selecting, between the condyles 9, 9' of the femur 4, the corresponding portion of the axoidal surfaces S1 and S2 of the motion.

This is the preferred position because it is in the femur 4 that we have the above mentioned cavity 12 extending between the two condyles 9, 9' of the femur 4 itself very close to the axoids S1, S2 of the spherical motion which the real motion of the joint 2 is approximated to.

Selecting the contact surfaces 10a, 11a exclusively in this position minimizes the work required on the bones, on both the femur 4 and on the tibia 5.

As better illustrated in Figure 6, means 13 for connecting the two surfaces 10a and 11a are also provided at this position.

These connecting means 13 comprise flexible elements identical in form and function to those described in patent application WO2007/074387 and described briefly below.

The connecting means 13 comprise a series of flexible elements 14, 15, and 16, preferably of laminar type.

The elements 14, 15, 16 are fastened to the guide surfaces 10a, 11a and are also located at the cavity 12.

In particular, the element 14 is fixed, substantially at a first end of it, to a portion of the component 11 at the surface 11a.

The element 14 is also fixed, substantially at a second end of it, to a portion of the component 10 at the surface 10a.

The element 14 is positioned partly on the surface 11a, from the portion of the component 11 it is fastened to up to the axis of instantaneous rotation at the given instant, and partly envelops the surface 10a, from the axis of instantaneous rotation at the given instant up to the portion of the component 10 it is fastened to.

As the tibia 5 moves relative to the femur 4, the flexible element 14 is unwrapped from the surface 10a and is wrapped onto the surface 11 a or vice versa.

The element 15 is fixed, substantially at a first end of it, to a second portion of the component 10 at the surface 10a.

The element 15 is also fixed, substantially at a second end of it, to a second portion of the component 11 at the surface 11a.

The element 15 is wrapped partly on the surface 10a, from the portion of the component 10 it is fastened to up to the axis of instantaneous rotation at the given instant, and partly on the surface 11a, from the axis of instantaneous rotation at the given instant up to the portion of the component 11 it is fastened to.

As the tibia 5 moves relative to the femur 4, the flexible element 15 is unwrapped from the surface 10a and is wrapped onto the surface 11 a or vice versa.

Preferably, the flexible element 15 is wrapped onto the guide surfaces 10a, 11a in the same way as the flexible element 14.

The flexible element 16 is positioned symmetrically to the flexible element 14 with respect to the flexible element 15.

The element 16 is fixed, substantially at a first end of it, to a portion of the component 11 at the surface 11a on the side opposite the flexible element 14 with respect to the flexible element 15.

The element 16 is also fixed, substantially at a second end of it, to a portion of the component 10 at the surface 10a on the side opposite the flexible element 14 with respect to the flexible element 15.

The element 16 is positioned partly on the surface 11a, from the portion of the component 11 it is fastened to up to the axis of instantaneous rotation at the given instant, and partly envelops the surface 10a, from the axis of instantaneous rotation at the given instant up to the portion of the component 10 it is fastened to.

As the tibia 5 moves relative to the femur 4, the flexible element 16 is unwrapped from the surface 10a and is wrapped onto the surface 11 a or vice versa.

The elements 14, 15 and 16 positioned in this way allow the surfaces 10a and 11a, that is to say, the components 10 and 11 of the prosthesis 1 to roll on each other without sliding.

Advantageously, the guide surfaces 10a, 11a that roll on each other without sliding are also suitably shaped to take into account the non-zero thickness of the flexible elements 14, 15, 16.

It should be noticed that in this configuration three flexible elements are necessary because the centre point "O" of the spherical motion is virtual, that is to say, it is not physically defined by means of a spherical coupling as in the previous embodiment and in the one described in more detail below.

The presence of three flexible elements thus allows the surfaces 10a and 11a, that is, the components 10 and 11 of the prosthesis 1 to roll on each other without sliding and, at the same time, prevents relative rotation of the components 10 and 11 about the common normal of the contact surfaces 10a and 11a.

As illustrated in particular in Figure 5, the components 9 and 10 comprise working or load surfaces 10b and 11b in contact with each other and substantially the same as those described above.

The presence of the load surfaces 10b and 11 b is particularly advantageous because the surfaces 10a and 11a, which serve to guide the joint 2, preferably have a reduced medio-lateral extension.

Preferably, as mentioned, the load surfaces 10b, 11b are constructed using the conic axoidal surfaces S1, S2 of the motion.

More specifically, the surfaces 10b, made in the form of replicas of the natural condyles, are attached to the axoidal surface S1.

The rotation of the surface S1 on the surface S2, being axoids of the motion, generates the surfaces 11b associated with the surface S2 itself.

Alternatively, the load surfaces 10b are preferably in the form of portions of conic surfaces with curvilinear directrix with vertex at "O", approximating the natural condyles.

Advantageously, the load surfaces 11b generated using the procedure described above are also portions of conic surfaces with curvilinear directrix with vertex at "O".

The mating load surfaces 10b, 11b therefore serve mainly to transmit the forces between the bone segments 4, 5.

Looking at Figure 5 from left to right, the prosthesis 1 component 10 associated with the femur 4 thus has a first part comprising the load surface 10b, a central part constituted by the surface 10a defined as a portion of the axoidal surface S1 of the spherical motion, and a third part comprising another load surface 10b.

In symmetrical fashion, again looking at Figure 5 from left to right, the prosthesis 2 component 11 associated with the tibia has a first portion modelled as mating surface 11b of the first load surface 10b of the first component 10, a central part modelled as portion of the axoid S2 of the spherical motion, and a third portion modelled as mating surface 11b of the second load surface 10b of the component 10.

In practice, in this embodiment, the relative motion of the components 10 and 11 occurs as if the components were constrained by a spherical pair and as if the relative motion were constrained by the surfaces 10a and 11a, which constitute guide surfaces that leave the device 1 with only one degree of freedom.

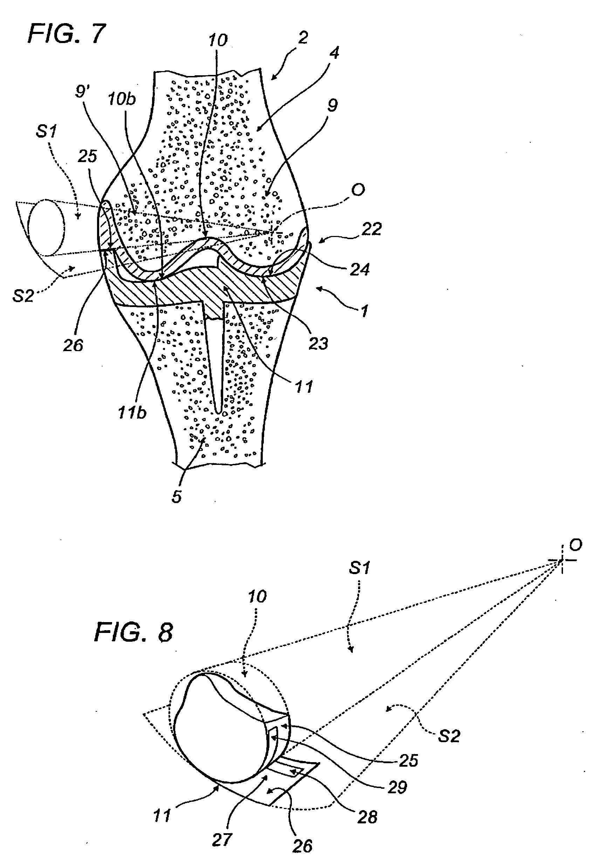

Figure 7 shows a fourth preferred embodiment of the orthopaedic device 1 according to the invention. In the fourth preferred embodiment, the orthopaedic device 1 according to the invention comprises, again, a first component 10 designed to be rigidly coupled to the femur 4 and a second component 11 designed to be rigidly coupled to the tibia 5.

The device 1 comprises a spherical coupling, that is to say, a spherical pair 22 centred at the fixed point "O" of the spherical motion located in the condyle 9 of the femur 4.

In the preferred embodiment illustrated by way of example, the spherical pair 22 comprises a spherical socket 23 formed in the component 11 and in which a spherical portion 24 formed on the first component 10 moves.

In other words, the spherical pair 22 comprises the spherical, convex portion 24 associated with the femur 4 and a corresponding concave portion of the spherical surface 23 associated with the tibia 5 where the first spherical surface 24 is engaged in the second spherical surface 23 to form the spherical pair 22.

For guiding and constraining the spherical pair 22, and hence the device 1, the latter comprises a second pair of surfaces 25, 26, the former associated with the femur 4 and the latter associated with the tibia 5.

The mating surfaces 25, 26 are portions of the above mentioned conic surfaces S1, S2, which are the axoidal surfaces of the spherical motion suitably replicated in the zone concerned.

In practice, the component 10 comprises the guide surface 25 and the component 11 comprises the guide surface 26 that mates with the surface 25.

In other terms, the guide surfaces 25, 26 fulfil the same function as the guide surfaces 10a, 11a of the embodiment illustrated in Figure 5.

Advantageously, the surfaces 25, 26 are located laterally in the condyle 9', remaining preferably within the natural dimensions of the knee 3.

As illustrated schematically in particular in Figure 8, the conic surfaces 25, 26 are constrained to roll on each other by the connecting means 27.

The connecting means 27 comprise a pair of flexible elements 28, 29.

The flexible elements 28, 29 are positioned in such a way as to allow the surfaces 25 and 26 to roll on each other without sliding.

It should be noted that this embodiment comprises only one pair of flexible elements since the bone segments 4, 5 are constrained to each other also by the spherical pair 22.

The elements 28, 29, are fastened to the guide surfaces 25, 26 and are also located laterally in the condyle 9'.

In particular, the element 28 is fixed, substantially at a first end of it, to a portion of the component 11 at the axoidal surface 26.

The element 28 is also fixed, substantially at a second end of it, to a second portion of the component 10 at the axoidal surface 25.

The element 28 is positioned partly on the surface 26, from the portion of the component 11 it is fastened to up to the axis of instantaneous rotation at the given instant, and partly envelops the surface 25, from the axis of instantaneous rotation at the given instant up to the portion of the component 10 it is fastened to.

As the tibia 5 moves relative to the femur 4, the flexible element 28 is unwrapped from the surface 25 and is wrapped onto the surface 26 or vice versa.

The element 29 is fixed, substantially at a first end of it, to a second portion of the component 10 at the surface 25.

The element 29 is also fixed, substantially at a second end of it, to a second portion of the component 11 at the surface 26.

The element 29 is wrapped partly on the surface 25, from the portion of the component 10 it is fastened to up to the axis of instantaneous rotation at the given instant, and partly on the surface 26, from the axis of instantaneous rotation at the given instant up to the portion of the component 11 it is fastened to.

As the tibia 5 moves relative to the femur 4, the flexible element 28 is unwrapped from the surface 25 and is wrapped onto the surface 26 or vice versa.

Preferably, the flexible element 29 is wrapped onto the guide surfaces 25, 26 in the same way as the flexible element 28.

Preferably, the guide surfaces 25 and 26 and the flexible connecting means 27 are located on the condyle 9' side while the spherical pair 22 is located at the first, medial condyle 9.

The orthopaedic device 1 illustrated in Figure 7 comprises a pair of mating load surfaces 10b, 11b.

The surfaces 10b, 11b are preferably located at the condyle 9' and made in the form of mating surfaces.

Preferably, as mentioned in connection with other embodiments, the surface 10b is made to recreate the femoral condyle and the corresponding surface 11b is generated as mating profile of the surface 10b based on the spherical motion that approximates the real spatial motion of the knee 3.

Alternatively, the load surfaces 10b are preferably in the form of portions of conic surfaces with curvilinear directrix with vertex at "O", approximating the natural condyles.

Advantageously, the load surfaces 11b generated using the procedure described above are also portions of conic surfaces with curvilinear directrix with vertex at "O".

In practice, in this embodiment, the relative motion of the components 10 and 11 is defined by the spherical pair 22 and is constrained by the surfaces 25 and 26 in such a way as to leave the device 1 with only one degree of freedom.

Used as second implants or revision implants, the third and fourth embodiments described above are extremely advantageous.

In some cases, in the event of primary prosthesis failure or of serious joint destruction, it is in fact necessary to use prostheses that are more constrained, to be implanted in a second operation to replace the first prosthesis with a second one.

The invention described above is susceptible of industrial application and may be modified and adapted in several ways without thereby departing from its scope as defined by the appended claims.

## Claims

1. An orthopaedic device for the mobile connection of a first and a second bone segment (4, 5) forming a joint (2), the device comprising: a first component (10) that can be associated with the first bone segment (4); and a second component (11) that can be associated with the second bone segment (5), means (13, 17, 22, 27) for connecting the first component (10) and the second component (11) in order to join the first and second components (10, 11) to each other; and means (10a, 11 a, 25, 26,) for constraining the relative motion between the first component (10) and the second component (11); **characterized in that** the first and second components (10, 11) are coupled virtually or physically through the connecting means (13, 17, 22, 27,) and the constraining means (10a, 11a, 25, 26,) by a spherical kinematic pair (17, 22) centred at a point (O) that is the centre of a spherical motion that approximates the real relative spatial motion between the first bone segment (4) and the second bone segment (5), the constraining means (10a, 11a, 25, 26,) limiting the spherical kinematic pair to one degree of freedom, wherein the spherical motion is defined by a model (100) comprising a first member coupled to a second member by a spherical kinematic pair (6) and by a first and a second rod (7, 8) representing two natural anatomical ligamentous structures of said joint (2) for connecting the first member and the second member, the spherical kinematic pair (6) of the model (100), the first rod (7) and the second rod (8) defining an axis (R0) of instantaneous rotation, the geometric locus of the positions occupied by the axis (R0) of instantaneous rotation during motion being a first ruled surface (S1) with curvilinear directrix for the first member (4) and a second ruled surface (S2) with curvilinear directrix for the second member (5), the first and the second ruled surfaces (S1, S2) with curvilinear directrix being the axoids of the spherical motion.

2. The device according to claim 1, wherein the first component (10) has at least one first load surface (10b), the second component (11) having a second load surface (11b) which mates with the first load surface (10b) in the spherical motion that approximates the real relative spatial motion between the first bone segment (4) and the second bone segment (5).

3. The device according to claim 2, wherein the first load surface (10b) is located substantially at a condyle (9) of the first bone segment (4).

4. The device according to any of the foregoing claims, wherein the first component (10) has a third load surface (10b) at a second condyle (9') of the first bone segment (4), the second component (11) having a fourth load surface (11b) which mates with the third load surface (10b) in the spherical motion that approximates the real relative spatial motion between the first bone segment (4) and the second bone segment (5).

5. The device according to any of the foregoing claims, wherein the first load surface (10b) replicates a condyle (9) of the first bone segment (4).

6. The device according to any of the foregoing claims, wherein the third load surface (10b) replicates a second condyle (9') of the first bone segment (4).

7. The device according to claim 2, wherein at least one of the first or third load surface (10b) is in the form of a portion of a conic surface with curvilinear directrix with vertex at the centre point (O) of the spherical motion approximating the real relative spatial motion between the first bone segment (4) and the second bone segment (5).

8. The device according to claim 7, wherein the load surface (11b) that mates with the first or third load surface (10b) is in the form of a portion of a conic surface with curvilinear directrix with vertex at the centre point (O) of the spherical motion approximating the real relative spatial motion between the first bone segment (4) and the second bone segment (5).

9. The device according to any of the foregoing claims, wherein the constraining means (10a, 11a, 25, 26,) comprise guide rods (20, 21) operating between the first component (10) and the second component (11).

10. The device according to claim 9, wherein the first and second rods (20, 21) are positioned in the same way as the cruciate ligaments of a knee joint, the first and second bone segments (4, 5) being constituted by the femur (4) and the tibia (5), respectively.

11. The device according to claim 10, wherein the first and second rods (20, 21) are constituted by the cruciate ligaments.

12. The device according to any of the foregoing claims from 9 to 11, wherein the guide rods (20, 21) are constituted by natural, artificial or biological ligament portions.

13. The device according to any of the foregoing claims, wherein the connecting means (13, 17, 22, 27,) comprise a spherical pair (17, 22) for coupling the first component (10) to the second component (11), the spherical pair (17, 22) having its centre at the fixed point (O).

14. The device according to claim 13, wherein the first component (10) comprises a spherical portion (19, 24) and the second component (11) comprises a spherical socket (18, 24), the spherical portion (19, 24) being constrained to move within the spherical socket (18, 24).

15. The device according to any of the foregoing claims, wherein the first and second components (10, 11) comprise, respectively, first and second mating guide surfaces (10a, 11a, 25, 26) in the form of portions of the first and second ruled surfaces (S1, S2) with curvilinear directrices and constituting axoids of the spherical motion centred at the fixed point (O), the spherical motion approximating the real relative spatial motion between the first bone segment (4) and the second bone segment (5), the first and second guide surfaces (10a, 11a, 25, 26) at least partly constituting the constraining means (10a, 11a, 25, 26).

16. The device according to claim 15, wherein the connecting means (13, 17, 22, 27) comprise at least a first and a second flexible element (14, 15, 28, 29) fastened to the first and second components (10, 11) at the first and second guide surfaces (10a, 11a).

17. The device according to claim 15 or 16, wherein the first and second guide surfaces (10a, 11a) are located substantially at a central portion of the first and second components (10, 11), that is to say, between a first and a second condyle (9, 9') of the bone segment (4).

18. The device according to claim 15 or 16, wherein the first and second guide surfaces (10a, 11a) are located at a lateral end of the first and second components (10, 11).

19. The device according to any of the foregoing claims, wherein the centre point (O) of a spherical motion approximating the real relative spatial motion between the first bone segment (4) and the second bone segment (5) is located in the medial condyle (9) of the femur (4), the first and second bone segments (4, 5) being constituted by the femur (4) and the tibia (5), respectively.

20. The device according to any of the foregoing claims, wherein the centre point (O) of a spherical motion approximating the real relative spatial motion between the first bone segment (4) and the second bone segment (5) is located in a medial position relative to the sagittal midplane of a knee, the first and second bone segments (4, 5) being constituted by the femur (4) and the tibia (5), respectively.

## Patentansprüche

1. Orthopädische Vorrichtung für die mobile Verbindung eines ersten und eines zweiten Knochensegments (4, 5), bildend ein Gelenk (2), wobei die Vorrichtung umfasst: eine erste Komponente (10), die mit dem ersten Knochensegment (4) assoziiert werden kann; eine zweite Komponente (11), die mit dem zweiten Knochensegment (5) assoziiert werden kann; Mittel (13, 17, 22, 27) zum Verbinden der ersten Komponente (10) und der zweiten Komponente (11), um die erste und die zweite Komponente (10, 11) zusammenzufügen; Mittel (10a, 11a, 25, 26), um die relative Bewegung zwischen der ersten Komponente (10) und der zweiten Komponente (11) zu hemmen, **dadurch gekennzeichnet, dass** die erste Komponente und die zweite Komponente (10, 11) virtuell oder materiell durch die Verbindungsmittel (13, 17, 22, 27) und die Hemmmittel (10a, 11a, 25, 26) durch ein sphärisches kinematisches Paar (17, 22) gekoppelt sind, zentriert an einem Punkt (0), der die Mitte einer sphärischen Bewegung ist, die die relative wirkliche räumliche Bewegung zwischen dem ersten Knochensegment (4) und dem zweiten Knochensegment (5) approximiert, wobei die Hemmmittel (10a, 11a, 25, 26) das sphärische kinematische Paar auf einen Freiheitsgrad begrenzen, wobei die sphärische Bewegung durch ein Modell (100) definiert wird, umfassend ein erstes Element, das mit einem zweiten Element durch ein sphärisches kinematisches Paar (6) und durch eine erste und eine zweite Stange (7, 8) gekoppelt ist, darstellend zwei natürliche anatomische bandförmige Strukturen des Gelenks (2), um das erste Element und das zweite Element zu verbinden, wobei das sphärische kinematische Paar (6) des Modells (100), die erste Stange (7) und die zweite Stange (8) eine Achse (R0) mit Momentandrehung definieren, wobei der geometrische Ort der von der Achse (R0) mit Momentandrehung während der Bewegung eingenommenen Positionen eine erste schraffierte Oberfläche (S1) mit kurvenförmiger Leitlinie für das erste Element (4) und eine zweite schraffierte Oberfläche (S2) mit kurvenförmiger Leitlinie für das zweite Element (5) ist, wobei die erste und die zweite schraffierte Oberfläche (S1, S2) mit kurvenförmiger Leitlinie die Axoiden der sphärischen Bewegung sind.

2. Vorrichtung nach Anspruch 1, wobei die erste Komponente (10) mindestens eine erste Ladeoberfläche (10b) aufweist und die zweite Komponente (11) eine zweite Ladeoberfläche (11b) aufweist, die passend zur ersten Ladeoberfläche (10b) in der sphärischen Bewegung ausgebildet ist, die die wirkliche relative räumliche Bewegung zwischen dem ersten Knochensegment (4) und dem zweiten Knochensegment (5) approximiert.

3. Vorrichtung nach Anspruch 2, wobei die erste Ladeoberfläche (10b) im Wesentlichen an einem Kondylus (9) des ersten Knochensegments (4) befindlich ist.

4. Vorrichtung nach einem der vorhergehenden Ansprüche, wobei die erste Komponente (10) eine dritte Ladeoberfläche (10b) an einem zweiten Kondylus (9') des ersten Knochensegments (4) aufweist und die zweite Komponente (11) eine vierte Ladeoberfläche (11b) aufweist, die passend zur dritten Ladeoberfläche (10b) in der sphärischen Bewegung ausgebildet ist, die die wirkliche relative räumliche Bewegung zwischen dem ersten Knochensegment (4) und dem zweiten Knochensegment (5) approximiert.

5. Vorrichtung nach einem der vorhergehenden Ansprüche, wobei die erste Ladeoberfläche (10b) einen Kondylus (9) des ersten Knochensegments (4) reproduziert.

6. Vorrichtung nach einem der vorhergehenden Ansprüche, wobei die dritte Ladeoberfläche (10b) einen zweiten Kondylus (9') des ersten Knochensegments (4) reproduziert.

7. Vorrichtung nach Anspruch 2, wobei mindestens entweder die erste oder dritte Ladeoberfläche (10b) in der Form eines Abschnitts einer konischen Oberfläche mit kurvenförmiger Leitlinie mit Scheitelpunkt am mittigen Punkt (0) der sphärischen Bewegung, die die wirkliche relative räumliche Bewegung zwischen dem ersten Knochensegment (4) und dem zweiten Knochensegment (5) approximiert, ausgebildet ist.

8. Vorrichtung nach Anspruch 7, wobei die Ladeoberfläche (11b), die passend zur ersten oder dritten Ladeoberfläche (10b) ausgebildet ist, in der Form eines Abschnitts einer konischen Oberfläche mit kurvenförmiger Leitlinie mit Scheitelpunkt am mittigen Punkt (0) der sphärischen Bewegung, die die wirkliche relative räumliche Bewegung zwischen dem ersten Knochensegment (4) und dem zweiten Knochensegment (5) approximiert, ausgebildet ist.

9. Vorrichtung nach einem der vorhergehenden Ansprüche, wobei die Hemmmittel (10a, 11a, 25, 26) Führungsstangen (20, 21) umfassen, die zwischen der ersten Komponente (10) und der zweiten Komponente (11) arbeiten.

10. Vorrichtung nach Anspruch 9, wobei die erste und die zweite Stange (20, 21) auf dieselbe Weise wie die Kreuzbänder eines Kniegelenks positioniert sind, wobei das erste und das zweite Knochensegment (4, 5) jeweils aus dem Femur (4) und der Tibia (5) bestehen.

11. Vorrichtung nach Anspruch 10, wobei die erste und die zweite Stange (20, 21) aus den Kreuzbändern bestehen.

12. Vorrichtung nach einem der vorhergehenden Ansprüche 9 bis 11, wobei die Führungsstangen (20, 21) aus natürlichen, künstlichen oder biologischen Bandabschnitten bestehen.

13. Vorrichtung nach einem der vorhergehenden Ansprüche, wobei die Verbindungsmittel (13, 17, 22, 27) ein sphärisches Paar (17, 22) zum Kuppeln der ersten Komponente (10) mit der zweiten Komponente (11) umfassen, wobei sich der Mittelpunkt des sphärischen Paars (17, 22) am fixierten Punkt (0) befindet.

14. Vorrichtung nach Anspruch 13, wobei die erste Komponente (10) einen sphärischen Abschnitt (19, 24) und die zweite Komponente (11) einen sphärischen Sockel (18, 24) umfasst, wobei der sphärische Abschnitt (19, 24) eingeschränkt ist, um sich innerhalb des sphärischen Sockels (18, 24) zu bewegen.

15. Vorrichtung nach einem der vorhergehenden Ansprüche, wobei die erste und die zweite Komponente (10, 11) jeweils eine erste und eine zweite passende Führungsoberfläche (10a, 11a, 25, 26) in der Form von Abschnitten der ersten und der zweiten schraffierten Oberfläche (S1, S2) mit kurvenförmigen Leitlinien aufweisen und Axoiden der sphärischen Bewegung darstellen, zentriert am fixierten Punkt (0), wobei die sphärische Bewegung die wirkliche relative räumliche Bewegung zwischen dem ersten Knochensegment (4) und dem zweiten Knochensegment (5) approximiert, wobei die erste und die zweite Führungsoberfläche (10a, 11a, 25, 26) mindestens teilweise die Hemmmittel (10a, 11a, 25, 26) darstellen.

16. Vorrichtung nach Anspruch 15, wobei die Verbindungsmittel (13, 17, 22, 27) mindestens ein erstes und ein zweites flexibles Element (14, 25, 28, 29) umfassen, befestigt an der ersten und der zweiten Komponente (10, 11) an der ersten und der zweiten Führungsoberfläche (10a, 11a).

17. Vorrichtung nach Anspruch 15 oder 16, wobei die erste und die zweite Führungsoberfläche (10a, 11a) im Wesentlichen an einem mittigen Abschnitt der ersten und der zweiten Komponente (10, 11) platziert sind, d. h. zwischen einem ersten und einem zweiten Kondylus (9, 9') des Knochensegments (4).

18. Vorrichtung nach Anspruch 15 oder 16, wobei die erste und die zweite Führungsoberfläche (10a, 11a) an einem seitlichen Ende der ersten und der zweiten Komponente (10, 11) befindlich sind.

19. Vorrichtung nach einem der vorhergehenden Ansprüche, wobei der mittige Punkt (0) einer sphärischen Bewegung, die die wirkliche relative räumliche Bewegung zwischen dem ersten Knochensegment (4) und dem zweiten Knochensegment (5) approximiert, im mittleren Kondylus (9) des Femurs (4) befindlich ist, wobei das erste und das zweite Knochensegment (4, 5) jeweils aus dem Femur (4) und der Tibia (5) bestehen.

20. Vorrichtung nach einem der vorhergehenden Ansprüche, wobei der mittige Punkt (0) einer sphärischen Bewegung, die die wirkliche relative räumliche Bewegung zwischen dem ersten Knochensegment (4) und dem zweiten Knochensegment (5) approximiert, in einer mittigen Position relativ zur sagittalen Mittelebene eines Knies befindlich ist, wobei das erste und das zweite Knochensegment (4, 5) jeweils aus dem Femur (4) und der Tibia (5) bestehen.

## Revendications

1. Dispositif orthopédique destiné au raccordement mobile d'un premier et d'un second segment osseux (4, 5) formant une articulation (2), le dispositif comprenant : un premier composant (10) pouvant être associé au premier segment osseux (4) ; et un second composant (11) pouvant être associé au second segment osseux (5), des moyens (13, 17, 22, 27) servant à raccorder le premier composant (10) et le second composant (11) afin de joindre les premier et second composants (10, 11) l'un à l'autre ; et des moyens (10a, 11a, 25, 26) destinés à contraindre le mouvement relatif entre le premier composant (10) et le second composant (11) ; **caractérisé en ce que** les premier et second composants (10, 11) sont accouplés virtuellement ou physiquement par l'intermédiaire de moyens de raccordement (13, 17, 22, 27) et de moyens de contrainte (10a, 11a, 25, 26) par une paire cinématique sphérique (17, 22) centrée en un point (0) étant le centre d'un mouvement sphérique se rapprochant du mouvement réel correspondant dans l'espace entre le premier segment osseux (4) et le second segment osseux (5), les moyens de contrainte (10a, 11a, 25, 26) limitant la paire cinématique sphérique à un degré de liberté, dans lequel le mouvement sphérique est défini par un modèle (100) comprenant un premier membre accouplé à un second membre par une paire cinématique sphérique (6) et par une première et une seconde tige (7, 8) représentant deux structures ligamenteuses anatomiques naturelles de ladite articulation (2) pour relier le premier membre et le second membre, la paire cinématique sphérique (6) du modèle (100), la première tige (7) et la seconde tige (8) définissant un axe (R0) de rotation instantanée, le lieu géométrique des positions occupées par l'axe (R0) de rotation instantanée lors du mouvement étant une première surface réglée (S1) à directrice curviligne pour le premier membre (4) et une seconde surface réglée (S2) à directrice curviligne pour le second membre (5), les première et seconde surfaces réglées (S1, S2) à directrice curviligne étant les axoïdes du mouvement sphérique.

2. Dispositif selon la revendication 1, dans lequel le premier composant (10) possède au moins une première surface de charge (10b), le second composant (11) ayant une seconde surface de charge (11b) qui correspond à la première surface de charge (10b) dans le mouvement sphérique se rapprochant du mouvement réel correspondant dans l'espace entre le premier segment osseux (4) et le second segment osseux (5).

3. Dispositif selon la revendication 2, dans lequel la première surface de charge (10b) est située substantiellement au niveau d'un condyle (9) du premier segment osseux (4).

4. Dispositif selon l'une quelconque des revendications précédentes, dans lequel le premier composant (10) possède une troisième surface de charge (10b) au niveau d'un second condyle (9') du premier segment osseux (4), le second composant (11) ayant une quatrième surface de charge (11b) qui correspond à la troisième surface de charge (10b) dans le mouvement sphérique se rapprochant du mouvement réel correspondant dans l'espace entre le premier segment osseux (4) et le second segment osseux (5).

5. Dispositif selon l'une quelconque des revendications précédentes, dans lequel la première surface de charge (10b) reproduit un condyle (9) du premier segment osseux (4).

6. Dispositif selon l'une quelconque des revendications précédentes, dans lequel la troisième surface de charge (10b) reproduit un second condyle (9') du premier segment osseux (4).

7. Dispositif selon la revendication 2, dans lequel au moins l'une des première ou troisième surfaces de charge (10b) a la forme d'une partie d'une surface conique à directrice curviligne ayant le sommet au centre (0) du mouvement sphérique se rapprochant du mouvement réel correspondant dans l'espace entre le premier segment osseux (4) et le second segment osseux (5).

8. Dispositif selon la revendication 7, dans lequel la surface de charge (11b) correspondant à la première ou à la troisième surface de charge (10b) a la forme d'une partie d'une surface conique à directrice curviligne ayant le sommet au centre (0) du mouvement sphérique se rapprochant du mouvement réel correspondant dans l'espace entre le premier segment osseux (4) et le second segment osseux (5).

9. Dispositif selon l'une quelconque des revendications précédentes, dans lequel les éléments de contrainte (10a, 11a, 25, 26) comprennent des tiges de guidage (20, 21) opérant entre le premier composant (10) et le second composant (11).

10. Dispositif selon la revendication 9, dans lequel les première et seconde tiges (20, 21) sont positionnées de la même façon que les ligaments croisés d'une articulation de genou, les premier et second segments osseux (4, 5) étant, respectivement, constitués par le fémur (4) et le tibia (5).

11. Dispositif selon la revendication 10, dans lequel les première et seconde tiges (20, 21) sont constituées par les ligaments croisés.

12. Dispositif selon l'une quelconque des revendications de 9 à 11, dans lequel les tiges de guidage (20, 21) sont constituées par des parties de ligaments naturel, artificiel ou biologique.

13. Dispositif selon l'une quelconque des revendications précédentes, dans lequel les moyens de raccordement (13, 17, 22, 27) comprennent une paire sphérique (17, 22) pour accoupler le premier composant (10) au second composant (11), la paire sphérique (17, 22) ayant son centre en correspondance du point fixe (O).

14. Dispositif selon la revendication 13, dans lequel le premier composant (10) comprend une partie sphérique (19, 24) et le second composant (11) comprend une cavité sphérique (18, 24), la partie sphérique (19, 24) étant contrainte pour se déplacer à l'intérieur de la cavité sphérique (18, 24).

15. Dispositif selon l'une quelconque des revendications précédentes, dans lequel les premier et second composants (10, 11) comprennent, respectivement, des première et seconde surfaces de guidage qui se correspondent (10a, 11a, 25, 26) ayant la forme de parties des première et seconde surfaces réglées (S1, S2) à directrices curvilignes et constituant les axoïdes du mouvement sphérique centré au niveau du point fixe (0), le mouvement sphérique se rapprochant du mouvement réel correspondant dans l'espace entre le premier segment osseux (4) et le second segment osseux (5), les première et seconde surfaces de guidage (10a, 11a, 25, 26) constituant au moins partiellement les moyens de contrainte (10a, 11a, 25, 26).

16. Dispositif selon la revendication 15, dans lequel les moyens de raccordement (13, 25 17, 22, 27) comprennent au moins un premier et un second élément flexible (14, 15, 28, 29) attachés aux premier et second composants (10, 11) au niveau des première et seconde surfaces de guidage (10a, 11a).

17. Dispositif selon la revendication 15 ou 16, dans lequel les première et seconde surfaces de guidage (10a, 11a) sont substantiellement situées en correspondance d'une partie centrale des premier et second composants (10, 11), autrement dit, entre un premier condyle et un second condyle (9, 9') du segment osseux (4).

18. Dispositif selon la revendication 15 ou 16, dans lequel les première et seconde surface de guidage (10a, 11a) sont situées en correspondance d'une extrémité latérale des premier et second composants (10, 11).

19. Dispositif selon l'une quelconque des revendications précédentes, dans lequel le centre (0) d'un mouvement sphérique se rapprochant du mouvement réel correspondant dans l'espace entre le premier segment osseux (4) et le second segment osseux (5) est situé dans le condyle interne (9) du fémur (4), les premier et second segments osseux (4, 5) étant, respectivement, constitués par le fémur (4) et le tibia (5).

20. Dispositif selon l'une quelconque des revendications précédentes, dans lequel le centre (0) d'un mouvement sphérique se rapprochant du mouvement réel correspondant dans l'espace entre le premier segment osseux (4) et le second segment osseux (5) est situé dans une position interne par rapport au plan sagittal médian d'un genou, les premier et second segments osseux (4, 5) étant, respectivement, constitués par le fémur (4) et le tibia (5).
